# FASCICULE DE BREVET EUROPEEN

(11) **EP 0 820 768 B1**
(45) Date de publication et mention de la délivrance du brevet: **10.04.2002**
(21) Numéro de dépôt: 97401735.2
(22) Date de dépôt: 18.07.1997
(51) Int. Cl.: A61K 7/48, A61K 7/06, A61K 7/40, A61K 7/42

(54) **Nouveaux dérivés de la mélatonine en tant qu'agent anti-radicaux libres et compositions les comprenant**
Neue Melatonin-Derivaten als Wirkstoff gegen freien Radikale sowie diese enthaltende Präparate
New melatonin derivatives as anti-free-radical agent and compositions comprising the same

(30) Priorité: 25.07.1996 FR 9609389
(43) Date de publication de la demande: 28.01.1998
(73) Titulaire: L'OREAL, 75008 Paris (FR)
(72) Inventeur: Nadaud, Jean-François, 92140 Clamart (FR); Colin, Christian, 75020 Paris (FR)
(74) Mandataire: Dodin, Catherine

(56) Documents cités:
- WO-A-97/06779
- REITER R. J.: "Interactions of the pineal hormone melatonin with oxygen-centered free radicals" BRAZILIAN J. MED. BIOL. RES., vol. 26, no. 11, 1993, pages 1141-55, XP000673603
- REITER,RUSSEL J. ET AL.: "melatonin in the context of the free radical theory of aging" ANN. N. Y. ACAD. SCI., vol. 786, 1996, pages 362-378, XP000673656
- HARDELAND R. ET AL.: "the significance of the metabolism of the neurohormone melatonin : antioxidative protection and formation of bioactive substances" NEUROSCI. BIOBEHAV. REV., vol. 17, no. 3, 1993, pages 347-357, XP000673658
- REITER J.: "the indoleamine melatonin as a free radical scavenger, electron donor and antioxidant" ADV. EXP. MED. BIOL., vol. 3398, 1996, pages 307-313, XP000673601
- RUSSEL J. REITER ET AL.: "melatonin, free radicals and cancer initiation" ADVANCES IN PINEAL RESEARCH, vol. 7, 1994, pages 211-228, XP000673655

## Description

La présente invention a pour objet de nouveaux dérivés de la mélatonine présentant une activité antiradicaux libres (ARL) améliorée et les compositions dermo-cosmétiques les comprenant.

Au cours du temps, il apparaît différents signes sur la peau et/ou sur le cuir chevelu et/ou sur les cheveux, très caractéristiques du vieillissement, se traduisant notamment par une modification de la structure et des fonctions cutanées.

Les principaux signes cliniques du vieillissement cutané sont notamment l'apparition de ridules et de rides profondes, en augmentation avec l'âge. Par ailleurs, le teint de la peau est généralement modifié et il peut exister sur certaines zones de la peau des irritations diffuses et parfois des télangiectasies. Un autre signe clinique du vieillissement est l'aspect sec et rêche de la peau qui est dû essentiellement à une desquamation plus importante. On constate enfin une perte de fermeté et de tonicité de la peau qui, comme pour les rides et les ridules, s'explique du moins en partie par une atrophie dermique et épidermique ainsi qu'un applatissement de la formation. On constate donc que les signes cliniques du vieillissement cutané résultent essentiellement d'un dysfonctionnement des principaux mécanismes biologiques intervenant au niveau de la peau.

Les radicaux libres sont connus parmi les facteurs responsables du vieillissement cellulaire, en particulier cutané. Ils proviennent en grande partie de l'oxygène moléculaire est sont induits par exemple par les polluants atmosphériques et/ou par le rayonnement ultraviolet.

On citera notamment les radicaux suivants:
- l'oxygène singulet, très oxydant, très toxique et d'une durée de vie très courte, produit de l'excitation de l'oxygène moléculaire par les photons de la lumière;
- le radical anion superoxyde, produit de l'addition d'un électron sur l'oxygène et pouvant donner lieu à la production des radicaux hydroxyles très réactifs;
- le radical hydroxyle, très oxydant et le plus toxique pour les cellules.

On citera en outre les radicaux lipo-peroxydes qui sont les produits d'oxydation des lipides membranaires et le fer extracellulaire qui, en réagissant avec le peroxyde d'hydrogène et le radical anion superoxyde accumulés en dehors de la cellule, va favoriser la production de radical hydroxyle.

Prévenir ou traiter les dommages causés par les radicaux libres sur la peau, le cuir chevelu ou les cheveux, en particulier le vieillissement cutané, qu'il soit intrinsèque ou extrinsèque, et les signes cliniques décrits auparavant, revient à maintenir ou améliorer l'apparence de la peau, du scalp ou des cheveux.

Différents produits ont été décrits comme possédant des propriétés ARL, ainsi que leur emploi en cosmétique. Il s'agit de composés comme les tocophérols, tels que la vitamine E, connus pour posséder, à la fois, des propriétés antioxydantes vis-à-vis des phospholipides de la membrane cellulaire, et des propriétés antiradicalaires (ARL) (J.B. CHAZAN et M.SZULC, *Cah. Nutr. Diet*., 1987, 6 XXII - 1, 66-76) ou les superoxyde-dismutases, par exemple extraites d'érythrocytes de boeuf (Markovitz, *J. Biol. Chem.*, 234, 1959, 40), d'*Escherichia coli* (Keele et Fridovich, *J. Biol. Chem*., 245, 1970, 6176) ou de souches bactériennes marines (FR 73.13670), ou encore la mélatonine.

La mélatonine, ou N-acétyl-5-méthoxytryptamine, surtout connue pour son activité circadienne régulant la production d'hormones, est également décrite pour son activité antioxydante (Reiter R J, *Verhandung der Deutschen Zoologischen Gesellschaft*, 87 (2), 1994, 195-204; Reiter R J & al., *Neuroendocrinoll Letter,* 15 (1-3), 1993, 103-113; Reiter R J & al., *J. Pineal Res.,* 18 (1), 1995, 1-11), en particulier son activité ARL (Reiter RJ & al., *Brazilian Journal of Médical and Biological Research,* 26 (22), 1993, 1141-1155). La mélatonine a également été décrite pour son utilisation en dermo-cosmétique pour améliorer l'apparence de la peau (JP 61 221 104; US 4 746 674), ou pour protéger la peau contre l'effet d'une irradiation aux rayonnements U.V. (EP 0 438 856; E. Bangha & al., Dermatology 191, [2], 176, 1995). La plupart des études de la mélatonine portent sur les phénomènes d'oxydation liés au vieillissement du cerveau (Poeggeler B & al., *J. Pineal Res.,* 14 (4), 1993, 151-168; Cagnoli C M & al., *J. Pineal Res.,* 18 (4), 1995, 222-226; Melchiorri D & al., *FASEB J.,* 9 (12), 1995, 1205-1210; Sewerynek E & al., *Neuroscience Letters,* 195 (3), 1995, 203-205) et il apparaît que la mélatonine est plus efficace que des ARL usuels tels que la vitamine E (Pieri C. & al., *Life Sciences,* 55 (15), 1994, 271-276).

La demanderesse a maintenant découvert que certains dérivés de la mélatonine présentent une activité antiradicaux libres améliorée par rapport à la mélatonine.

Il s'agit des dérivés de formule générale (I) 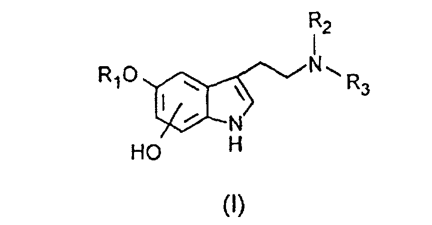 dans laquelle
R₁ représente un radical alkyle inférieur,
R₂ représente un atome d'hydrogène ou un radical alkyle inférieur, et
R₃ représente un atome d'hydrogène ou un radical acyle inférieur,
le radical hydroxyle sur le noyau indole pouvant se situer en position 4, 6 ou 7, ses sels, solvates ou bioprécurseurs physiologiquement acceptables.

D'une manière avantageuse, il s'agit des dérivés de formule générale (la) 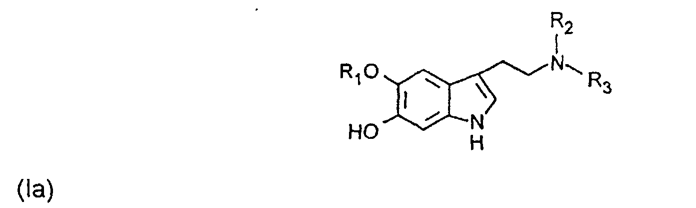 dans laquelle R₁, R₂ et R₃ sont définis ci-dessus et le radical hydroxyle est en position 6.

Par alkyle inférieur, on entend de préférence les radicaux alkyles en C₁ à C₄, linéaires ou ramifies, éventuellement substitués par un ou plusieurs halogènes (F, Cl ou Br). Il s'agit en particulier des radicaux méthyle, éthyle, propyle ou butyle. Cette définition s'applique au restes alkyles des radicaux acyles.

Par sel on entend tout sel d'addition d'un acide minéral ou organique physiologiquement acceptable, usuel pour des composés actifs en cosmétique ou en dermatologie, tels que des sels d'addition avec l'acide chlorhydrique, sulfurique, acétique, citrique, fumarique, hémisuccinique, maléique, etc.

Par bioprécurseur physiologiquement acceptable, on entend les dérivés aptes à libérer les composés de formule générale (I) ci-dessus une fois administrés en particulier les esters , tels que les alkyl phosphates, alkyl sulfates ou acyles (par exemple acétate), ou les osides (en particulier glucosyl, mannosyl, fructosyl, N-acétylglucamine, galactosyl) du radical hydroxyle.

D'une manière préférentielle, R₁ représente un radical méthyle, R₂ représente un atome d'hydrogène, et R₃ représente un radical acétyle.

La présente invention concerne donc l'utilisation cosmétique des dérivés de la mélatonine de formule générale (I) définie ci-dessus, à titre d'agent antiradicaux libres dans une composition cosmétique pour améliorer l'apparence de la peau, du scalp ou des cheveux.

La composition est de préférence une composition cosmétique pour lutter contre les dommages causés par les radicaux libres sur la peau, le scalp ou les cheveux et la présente invention concerne également un procédé de traitement cosmétique consistant à améliorer l'apparence de la peau, du scalp ou des cheveux, qui consiste à appliquer par voie topique sur la peau et/ou sur le cuir chevelu et/ou sur les cheveux, une composition comprenant à titre d'agent antiradicaux libres un dérivé de la mélatonine tel que défini ci-dessus.

Toutefois, cette composition peut également être une composition dermatologique et dans ce cas, la présente invention concerne aussi l'utilisation des dérivés de la mélatonine tels que définis ci-dessus pour la fabrication d'une composition pharmaceutique destinée au traitement dermatologique des signes cliniques du vieillissement cutané liés aux dommages causés par les radicaux libres.

La présente invention concerne enfin les compositions cosmétiques pour améliorer l'apparence de la peau, du scalp ou des cheveux comprenant au moins un dérivé de la mélatonine défini ci-dessus à titre d'agent anti-radicaux libres et un support cosmétiquement acceptable.

Dans ces compositions, les dérivés de la mélatonine selon l'invention peuvent être utilisés de préférence en une quantité inférieure à 10 % en poids par rapport au poids total de la composition. D'une manière générale, la quantité de dérivés de la mélatonine est comprise entre 0,0001 % à 10 % en poids, en particulier en une quantité allant de 0,0005 % à 1 % en poids, plus particulièrement en une quantité inférieure à 0,1 %, de l'ordre de 0,01 %.

On notera toutefois que les dérivés de la mélatonine sont actifs en des quantités inférieures à 10⁻⁴ % en poids, jusqu'à 10⁻¹⁴ % en poids.

La composition dans laquelle se trouve les dérivés de la mélatonine, cosmétique ou dermatologique, peut se présenter sous toutes les formes galéniques à usage topique normalement utilisées. La composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Les quantités des différents constituants des compositions sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, etc.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique ou dermatologique peut contenir également des adjuvants habituels dans le domaine cosmétique ou dermatologique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans ces domaines, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Dans la mesure où ils n'interfèrent pas avec l'activité de la mélatonine, les compositions selon l'invention peuvent contenir d'autres actifs destinés notamment à la prévention et/ou au traitement des affections cutanées.

### Activité in vitro

L'activité de dérivés selon l'invention a été étudiée dans différents tests ARL, pour des solutions à 0,03 %, et comparée à celle de la mélatonine et celles d'antiradicaux libres de référence.

Pour le test "Hp-Squalène" antioxygène singulet ¹O₂ (C. Vever-Bizet & al., Photochemistry and Photobiology, Vo150 N°3, 1989, 321-325; L.P. Srivastava & al., Photobiochemistry and Photobiophysics, 11, 1986, 129-137), la 6-hydroxymélatonine présente un pourcentage d'inhibition proche de 78%, suppérieur à celui obtenu pour la mélatonine et les autres inhibiteurs de l'oxygène singulet de référence.

Les résultats de ce test anti ¹O₂ sont reportés sur le Tableau I ci-après.

**Tableau I**

| **Test Hp-Squalène** | |
|---|---|
| **COMPOSE** | **% D'INHIBITION** |
| 6-hydroxy-mélatonine | 78 % |
| quercetine | 72 % |
| cantaxanthine | 67 % |
| β-carotène | 62 % |
| rutine | 51 % |
| mélatonine | 45 % |
| astaxanthine | 34 % |

Exemples de compositions Les exemples suivants de compositions illustrent l'invention. Dans les compositions, les proportions indiquées sont des pourcentages en poids.

### Exemple 1: Crème de soin du visage (émulsion huile dans eau)

| | |
|---|---|
| 6-hydroxy-mélatonine | 0,01 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau qsp | 100 % |

### Exemple 2: Gel pour le soin du visage

| | |
|---|---|
| 6-hydroxy-mélatonine | 0,001 |
| Hydroxypropylcellulose (Klucel H vendu par la société Hercules) | 1,00 |
| Antioxydant | 0,05 |
| Isopropanol | 40,00 |
| Conservateur | 0,30 |
| Eau qsp | 100 % |

## Revendications

1. Utilisation cosmétique d'un dérivé de la mélatonine de formule générale (I) 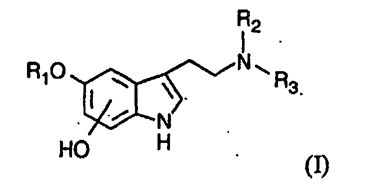 dans laquelle
R₁ représente un radical alkyle inférieur,
R₂ représente un atome d'hydrogène ou un radical alkyle inférieur, et
R₃ représente un atome d'hydrogène ou un radical acyle inférieur,
le radical hydroxyle sur le noyau indole pouvant se situer en position 4, 6 ou 7,
ses sels, solvates ou bioprécurseurs physiologiquement acceptables,
dans une composition cosmétique en tant qu'agent antiradicaux libres pour améliorer l'apparence de la peau, du scalp ou des cheveux.

2. Utilisation selon la revendication 1, **caractérisée en ce que** le radical hydroxyle est en position 6.

3. Utilisation selon l'une des revendications 1 ou 2, **caractérisée en ce que** R₁ représente un radical méthyle, R₂ représente un atome d'hydrogène, et R₃ représente un radical acétyle.

4. Utilisation selon l'une des revendications 1 à 3, **caractérisé en ce que** le dérivé de formule générale (I) est utilisé en en une quantité inférieure à 10 % en poids par rapport au poids total de la composition.

5. Utilisation selon la revendication 4, **caractérisée en ce que** la quantité de dérivés de la mélatonine est comprise entre 0,0001 % à 10 % en poids, en particulier en une quantité allant de 0,0005 % à 1 % en poids, plus particulièrement en une quantité inférieure à 0,1 %, de l'ordre de 0,01 %.

6. Procédé de traitement cosmétique pour améliorer l'apparence de la peau, du scalp ou des cheveux, **caractérisé en ce qu'**il consiste à appliquer par voie topique sur la peau et/ou sur le cuir chevelu et/ou sur les cheveux, une composition comprenant en tant qu'agent antiradicaux libres, un dérivé de la mélatonine de formule générale (I) définie dans l'une des revendications 1 à 3.

7. Utilisation des dérivés de la mélatonine de formule générale (I) définie dans l'une des revendications 1 à 3, pour la fabrication d'une composition pharmaceutique destinée au traitement dermatologique des signes cliniques du vieillissement cutané liés aux dommages causés par les radicaux libres.

8. Composition cosmétique pour améliorer l'apparence de la peau, du scalp ou des cheveux, **caractérisée en ce qu'**elle comprend, à titre d'agent anti-radicaux libres, au moins un dérivé de la mélatonine de formule générale (I) définie dans l'une des revendications 1 à 3, et un support cosmétiquement acceptable.

9. Composition selon la revendication 8, **caractérisée en ce que** la quantité de dérivés de la mélatonine est inférieure à 10 % en poids par rapport au poids total de la composition.

10. Composition selon la revendication 9, **caractérisée en ce que** la quantité de dérivés de la mélatonine est comprise entre 0,0001 % à 10 % en poids, en particulier en une quantité allant de 0,0005 % à 1 % en poids, plus particulièrement en une quantité inférieure à 0,1 %, de l'ordre de 0,01 %.

11. Composition selon la revendication 9, **caractérisée en ce que** la quantité de dérivés de la mélatonine est inférieure à 10⁻⁴ % en poids par rapport au poids total de la composition, jusqu'à 10⁻¹⁴ % en poids.

## Patentansprüche

1. Kosmetische Verwendung eines Melatoninderivats der allgemeinen Formel (I): 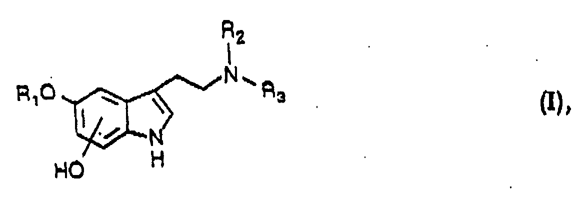 worin bedeuten:
R₁ eine niedere Alkylgruppe;
R₂ ein Wasserstoffatom oder eine niedere Alkylgruppe, und
R₃ ein Wasserstoffatom oder eine niedere Acylgruppe,
wobei sich die Hydroxygruppe in 4-, 6- oder 7-Stellung an dem Indolring befindet,
und ihrer Salze, Solvate oder Bioprecursoren, die physiologisch akzeptabel sind,
in einer kosmetischen Zusammensetzung als Radikal-Fänger für freie Radikale, um das Aussehen der Haut, der Kopfhaut oder der Haare zu verbessern.

2. Verwendung nach Anspruch 1, **dadurch gekennzeichnet, daß** sich die Hydroxygruppe in 6-Stellung befindet.

3. Verwendung nach einem der Ansprüche 1 oder 2, **dadurch gekennzeichnet, daß** die Gruppe R₁ Methyl bedeutet, die Gruppe R₂ ein Wasserstoffatom ist und die Gruppe R₃ Acetyl bedeutet.

4. Verwendung nach einem der Ansprüche 1 bis 3, dadurch gekennzeichhet, daß das Derivat der allgemeinen Formel (I) in einer Menge unter 10 Gew.-%, bezogen auf das Gesamtgewicht der Zusammensetzung, verwendet wird.

5. Verwendung nach Anspruch 4, **dadurch gekennzeichnet, daß** der Mengenanteil des Melatoninderivats im Bereich von 0,0001 bis 10 Gew.-%, insbesondere im Bereich von 0,0005 bis 1 Gew.-% und ganz besonders unter 0,1 % in der Größenordnung von 0,01 % liegt.

6. Verfahren zur kosmetischen Behandlung, um das Aussehen der Haut, der Kopfhaut oder der Haare zu verbessern, **dadurch gekennzeichnet, daß** es darin besteht, auf die Haut und/oder die Kopfhaut und/oder die Haare auf topischem Wege eine Zusammensetzung aufzubringen, die als Radikal-Fänger für freie Radikale ein Melatoninderivat der allgememen Formel (I) nach einem der Ansprüche 1 bis 3 enthält.

7. Verwendung von Melatoninderivaten der allgemeinen Formel (I) nach einem der Ansprüche 1 bis 3 zur Herstellung einer pharmazeutischen Zusammensetzung, die zur dermatologischen Behandlung von klinischen Anzeichen der Hautalterung vorgesehen ist, die mit Schäden zusammenhängen, die von freien Radikalen verursacht werden.

8. Kosmetische Zusammensetzung zur Verbesserung des Aussehens der Haut, der Kopfhaut oder der Haare, **dadurch gekennzeichnet, daß** sie als Radikal-Fänger für freie Radikale mindestens ein Melatoninderivat der allgemeinen Formel (I) nach einem der Ansprüche 1 bis 3 und einen kosmetisch akzeptablen Träger enthält.

9. Zusammensetzung nach Anspruch 8, **dadurch gekennzeichnet, daß** der Mengenanteil der Melatoninderivate unter 10 Gew.-%, bezogen auf das Gesamtgewicht der Zusammensetzung, liegt.

10. Zusammensetzung nach Anspruch 9, **dadurch, gekennzeichnet, daß** der Mengenanteil der Melatoninderivate im Bereich von 0,0001 bis 10 Gew.-%, insbesondere im Bereich von 0,0005 bis 1 Gew.-% und ganz besonders unter 0,1 % in der Größenordnung von 0,01 % liegt.

11. Zusammensetzung nach Anspruch 9, **dadurch gekennzeichnet, daß** der Mengenanteil der Melatoninderivate unter 10⁻⁴ Gew.-%, bezogen auf das Gesamtgewicht der Zusammensetzung, bis zu 10⁻¹⁴ Gew.-% liegt.

## Claims

1. Cosmetic use of a melatonin derivative of general Formula (I) 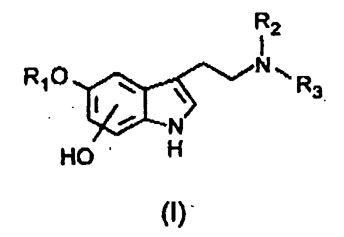 in which
R₁ represents a lower alkyl radical,
R₂ represents a hydrogen atom or a lower alkyl radical, and
R₃ represents a hydrogen atom or a lower acyl radical,
it being possible for the hydroxyl radical on the indole ring system to be in position 4, 6 or 7,
its physiologically acceptable salts, solvates or bioprecursors,
in a cosmetic combosition as an anti-free-radical agent for improving the appearance of the skin, the scalp or the hair.

2. Use according to Claim 1, **characterized in that** the hydroxyl radical is in position 6.

3. Use according to either of Claims 1 and 2, **characterized in that** R₁ represents a methyl radical, R₂ represents a hydrogen atom and R₃ represents an acetyl radical.

4. Use according to one of Claims 1 to 3, **characterized in that** the derivative of general formula (I) is used in an amount of less than 10% by weight relative to the total weight of the composition.

5. Use according to Claim 4, **characterized in that** the amount of melatonin derivatives is between 0.0001% and 10% by weight, in particular an amount ranging from 0.0005% to 1% by weight, more particularly an amount less than 0.1%, of about 0.01%.

6. Cosmetic treatment process for improving the appearance of the skin, the scalp or the hair, **characterized in that** it consists in applying topically to the skin and/or the scalp and/or the hair a composition comprising, as anti-free-radical agent, a melatonin derivative of general formula (I) defined in one of Claims 1 to 3.

7. Use of the melatonin derivatives of general formula (I) defined in one of Claims 1 to 3, for the manufacture of a pharmaceutical composition intended for the dermatological treatment of the clinical signs of ageing of the skin associated with damage caused by free radicals.

8. Cosmetic composition for improving the appearance of the skin, the scalp or the hair, **characterized in that** it comprises, as anti-free-radical agent, at least one melatonin derivative of general formula (I) defined in one of Claims 1 to 3, and a cosmetically acceptable support.

9. Composition according to Claim 8, **characterized in that** the amount of melatonin derivatives is less than 10% by weight relative to the total weight of the composition.

10. Composition according to Claim 9, **characterized in that** the amount of melatonin derivatives is between 0.0001% and 10% by weight, in particular an amount ranging from 0.0005% to 1% by weight, more particularly an amount less than 0.1%, of about 0.01%.

11. Composition according to Claim 9, **characterized in that** the amount of melatonin derivatives is less than 10⁻⁴% by weight relative to the total weight of the composition, down to 10⁻¹⁴% by weight.
